(19)

Europäisches
Patentamt
European
Patent Office
Office européen
des brevets

(11) **EP 2 657 254 A1**

(12) **EUROPEAN PATENT APPLICATION**
published in accordance with Art. 153(4) EPC

(43) Date of publication:
**30.10.2013  Bulletin 2013/44**

(21) Application number: **11850738.3**

(22) Date of filing: **26.12.2011**

(51) Int Cl.:
*C07K 17/00* (2006.01)     *C07K 17/04* (2006.01)
*C07K 17/06* (2006.01)     *C07K 17/12* (2006.01)
*G01N 30/54* (2006.01)     *G01N 30/88* (2006.01)

(86) International application number:
**PCT/JP2011/080065**

(87) International publication number:
**WO 2012/086837 (28.06.2012 Gazette 2012/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **24.12.2010   JP 2010288451**

(71) Applicants:
• **Asahi Kasei Medical Co., Ltd.
Chiyoda-ku
Tokyo 101-8101 (JP)**
• **Nomadic Bioscience Co., Ltd.
Okayama City, Okayama 701-1221 (JP)**

(72) Inventors:
• **KOGUMA, Ichiro
Tokyo 101-8101 (JP)**
• **SHIGEMATSU, Hiroki
Tokyo 101-8101 (JP)**
• **OKUYAMA, Kazuo
Tokyo 101-8101 (JP)**
• **SATO, Satoshi
Okayama-shi
Okayama 701-1221 (JP)**

(74) Representative: **von Kreisler Selting Werner
Deichmannhaus am Dom
Bahnhofsvorplatz 1
50667 Köln (DE)**

(54) **METHOD FOR IMMOBILIZING TEMPERATURE-RESPONSIVE PROTEIN A**

(57)     A method for manufacturing a carrier having temperature responsive protein A, which is protein A mutated such that a binding property to an antibody changes depending upon temperature, immobilized thereto, wherein the temperature responsive protein A has the binding property to the antibody in a first temperature region and no binding property to the antibody in a second temperature region; and the method comprises a step of immobilizing the temperature responsive protein A to a carrier surface at a temperature within the first temperature region in which the temperature responsive protein A has the binding property to the antibody.

Fig. 1

| Antibody binding temperature °C | 2 | 10 | 20 | 30 |
|---|---|---|---|---|
| Antibody binding amount mg／mL | 14.5 | 13.1 | 1.9 | 1.6 |

**Description**

Technical Field

The present invention relates to a method for immobilizing temperature responsive protein A, which is characterized by changing a binding property to an antibody depending upon temperature change, to a carrier.

Background Art

An antibody (immunoglobulin) is a physiologically active substance responsible for an immune response. Recently, availability of the antibody has been increased in applications such as medicines, diagnostic agents and separation/purification materials for the corresponding antigen protein. An antibody is taken from the blood of an immunized animal, a culture solution of a cell having antibody producibility or an ascitic fluid culture solution of the animal. However, such blood and a culture solution containing the antibody contain proteins other than the antibody or intricate contaminants derived from a raw-material solution used in the cell culture. Thus, to separate and purify the antibody from these impurity components, a complicated and time-consuming operation is usually required.

Liquid chromatography is important for separating and purifying an antibody. Examples of a chromatographic method for separating an antibody include gel filtration chromatography, affinity-chromatography, ion exchange chromatography and reverse phase chromatography. An antibody is separated and purified by a combination of these methods.

In the affinity-chromatography, a substance (ligand) having affinity for a target substance is immobilized to a carrier and allowed the target substance to specifically adsorb to the ligand to thereby separate the target substance from impurity components. Furthermore, a target substance adsorbed is eluted by reducing affinity for the ligand and collected. In this manner, the target substance is purified.

In the affinity-chromatography, an antibody having high purity and concentration is purified through the following (A) to (C) steps.

> (A) a step of loading a sample contaminated with impurities to a column (loading step)
> (B) a step of removing impurities except antibody to be purified from the column to which the sample is loaded (washing step)
> (C) a step of collecting the antibody to be purified from the column (elution step)

In the loading step and washing step, it is essential that the environment within the column is set such that the antibody to be purified strongly binds to an affinity ligand; however, in the elution step, the environment within the column is changed such that both substances are separated from each other. Conventionally, pH change is used for changing the environment.

As a ligand for affinity-chromatography for use in antibody purification, protein A derived from Staphylococcus and having extremely high specificity and affinity for the common region of antibodies and an antibody-binding domain of protein A are known and widely used in an antibody production step on an industrial scale.

However, affinity-chromatography using protein A or a part thereof as a ligand has problems ascribed to the nature of an antibody and its production is limited.

The problems are as follows. In order to elute the antibody to be purified from a column by pH change, the environment must be changed from a neutral region of pH 6 to 8 (pH of the loading and washing steps) where affinity of the antibody for protein A is high to an acidic region of pH 3 to 4 (pH of the elution step) where the affinity extremely decreases; however, in the acidic region, the antibody changes in configuration and causes aggregation/association, leading to malfunction (for example, see Patent Literature 1). As a result, the yield of separation/purification of the "antibody having its original nature" becomes extremely low.

Particularly, industrially the most useful humanized or human-type IgG used as a monoclonal-antibody medicine, since it has a higher affinity for protein A than other antibodies, requires use of a buffer having particularly strong acidity in elution time. Because of this, association/aggregation tends to occur in the elution time. Accordingly, inactivation of IgG becomes an industrial problem.

In the circumstances, to efficiently produce a highly purified antibody, development of a purification method without using an acidic environment in an elution step has been desired.

To solve the above problem, a purification method using protein A mutant (temperature responsive protein A), which changes a binding property to an antibody depending upon temperature, has been proposed (for example, see Patent Literature 2). According to the method proposed, a novel purification method requiring no pH change in an elution step can be provided by using temperature responsive protein A, which is capable of controlling loss and formation of its native configuration under conditions (pH 5 to 9, less than 60°C) where stability of configuration of the antibody to be purified is ensured without causing a failure such as damage, loss of function or occurrence of an unwanted function, as a ligand to be immobilized to a supporting medium for affinity-chromatography.

Citation List

Patent Literatures

Patent Literature 1: Japanese Patent Laid-Open No. 2005-206602
Patent Literature 2: WO2008/143199

Summary of Invention

Technical problem

Temperature responsive protein A is characterized by changing a binding property to an antibody depending upon temperature change; however, studies have not yet been made on an optimal method for immobilizing this to a supporting medium. Then, an object of the present invention is to provide an optimal method for immobilizing temperature responsive protein A characterized by changing a binding property to an antibody depending upon temperature change to a supporting medium.

Solution to Problem

The present inventors made research and development with a view of attaining the aforementioned object from various angles. As a result, we found that temperature responsive protein A, which is characterized by changing a binding property to an antibody depending upon temperature change, can be immobilized without losing its function by contacting the temperature responsive protein A with a coupling group immobilized to the surface of a carrier at the temperature, at which the temperature responsive protein A has an adsorptivity to an antibody, and covalently immobilizing the temperature responsive protein A to the carrier surface. The technique disclosed in the present invention cannot be totally expected from the prior art and development toward a novel separation system of an antibody, which has never been exist in the prior art, is expected. The present invention was accomplished based on such a finding.

Advantageous Effects of Invention

Based on the manufacturing method described in the present invention, a novel affinity chromatographic carrier can be manufactured. If such a carrier is used, useful physiologically active substances such as proteins including immunoglobulins will be successfully separated/purified on an industrial scale by changing temperature.

Brief Description of Drawings

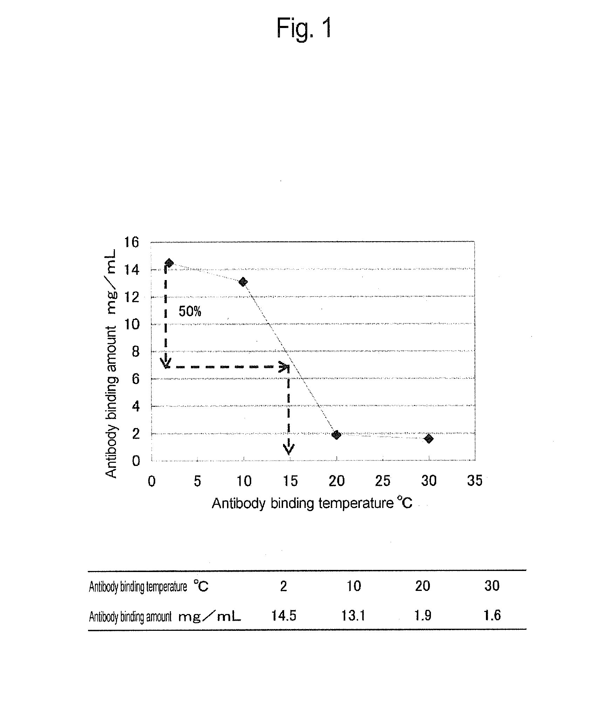

[Figure 1] Figure 1 shows a table and graph summarizing experimental results of adsorption/elution tests of an antibody by use of temperature responsive protein A immobilized to a carrier in accordance with the method described in Example 1.

Description of Embodiments

Now, an embodiment of the present invention (hereinafter, referred to as "the embodiment") will be described below in more detail. The embodiment relates to temperature responsive protein A, which is protein A mutated such that its binding property to an antibody changes depending upon temperature. Temperature responsive protein A has a binding property to an antibody in a first temperature region and does not have the binding property to the antibody in a second temperature region. The method for manufacturing a carrier having temperature responsive protein A immobilized thereto according to the embodiment include a step of immobilizing temperature responsive protein A to the surface of the carrier at a temperature within the first temperature region at which temperature responsive protein A has a binding property to an antibody.
The form of the supporting medium to be used as the carrier in the embodiment is not particularly limited and for example, membrane form such as flat membrane form and hollow fiber form or bead form can be used. A supporting medium of hollow fiber form is preferably used since it is easily molded into a module and the area of the membrane to be packed in a module container is large. Also, bead form is preferably used since it generally has a large surface area per volume compared to membrane form and a large amount of antibody can be adsorbed.

In the embodiment, the material for the supporting medium to be used as the carrier is not particularly limited. As the material for a membrane-form carrier, a polymer material that can be formed into a porous membrane is preferably used. Examples thereof that can be used include olefin resins such as polyethylene and polypropylene; polyester resins such as polyethylene terephthalate and polyethylene terenaphthalate; polyamide resins such as nylon 6 and nylon 66; fluorine-containing resins such as polyvinylidene fluoride and polychlorotrifluoro ethylene; and noncrystalline resins such as polystyrene, polysulfone, polyether sulfone and polycarbonate. Examples of the material for the bead-form carrier that can be used include glass, silica, a polystyrene resin, a methacrylic resin, crosslinked agarose, crosslinked dextran, crosslinked polyvinyl alcohol and crosslinked cellulose. Crosslinked polyvinyl alcohol and crosslinked cellulose can be preferably used since they have high hydrophilicity and can suppress adsorption of impurity components.

The supporting medium to be used in the embodiment has, for example, a plurality of pores. Although it is not particularly limited, the pore diameter is satisfactorily 5 to 1000 nm, preferably 10 to 700 nm and further preferably 20 to 500 nm. If the pore diameter is 5 nm or less, the molecular weight of antibody to be separated tends to reduce. In contrast, if the pore diameter is 1000 nm or more, the surface area of the supporting medium decreases, with the result that the binding capacity to an antibody tends to reduce.

In the embodiment, any coupling group may be introduced in the above supporting medium. Examples thereof include a carboxyl group activated by N-hydroxysuccinimide (NHS), a carboxyl group, a cyanogen bromide activated group, a hydroxide group, an epoxy group, an aldehyde group and a thiol group. Of the aforementioned coupling groups, an NHS activated carboxyl group, a carboxyl group, a cyanogen bromide activated group, an epoxy group and a formyl group, which are capable of coupling with a primary amino group, are preferable since temperature responsive protein A has the primary amino group. Particularly, the NHS activated carboxyl group is preferably used since it does not require other chemical agents in a coupling reaction and the reaction quickly proceeds to form a tight bond. The concentration (density) of the coupling groups immobilized to the supporting medium surface is preferably 50 $\mu$mol/mL or more and less than 1000 $\mu$mol/mL.

If the concentration of the coupling groups is less than 50 $\mu$mol/mL, the coupling efficiency of the coupling group with temperature responsive protein A tends to decrease.

If the concentration of the coupling groups is set at 50 $\mu$mol/mL or more, the coupling efficiency of the coupling group with temperature responsive protein A can be increased. In the embodiment, temperature responsive protein A is immobilized via a covalent bond to the supporting medium surface by contacting temperature responsive protein A with the coupling group immobilized to the surface of the supporting medium at the temperature at which temperature responsive protein A has an adsorptivity to an antibody. In this manner, temperature responsive protein A can be immobilized to the supporting medium surface without damaging function of temperature responsive protein A. Therefore, temperature responsive protein A can be immobilized to the carrier surface with a high coupling efficiency and without damaging the function of temperature responsive protein A by use of the supporting medium having the coupling groups at a high density. However, if the concentration of the coupling groups is 1000 $\mu$mol/mL or more, the number of bonds between the supporting medium and temperature responsive protein A increases and the activity of temperature responsive protein A tends to decrease.

The density of the coupling group can be measured by a method known to those skilled in the art. For example, in the case of an NHS activated carboxyl group, there are the HPLC method in which NHS is released from a carboxyl group with heat and alkali and the quantity of NHS is determined by the HPLC method; and a gravimetric method in which the weight (W1) of the carrier increased by an NHS activation reaction is measured.

In the embodiment, any method may be employed for introducing the coupling group into the supporting medium; however, a spacer is generally introduced between the supporting medium and the coupling groups. A method for introducing the coupling group is disclosed in various literatures.

In the embodiment, a graft polymer chain having the coupling group at a terminal and/or a side chain may be introduced into the supporting medium. The density of the coupling groups can be controlled, more specifically, enhanced at a discretion by introducing the graft polymer chain having the coupling group into the supporting medium. The graft polymer chain having the coupling group may be grafted to the supporting medium or the polymer chain having a precursor functional group that can be converted into the coupling group is grafted to the supporting medium and thereafter, the precursor functional group grafted may be converted into the coupling group.

As a method for introducing the graft polymer chain, any method may be employed. A polymer chain may be previously prepared and then coupled with the supporting medium. Furthermore, the graft chain can be directly polymerized on the supporting medium in accordance with a method such as a "living radical polymerization method" and a "radiation graft polymerization method". The "radiation graft polymerization method" is preferably used since a reaction initiator needs not to be introduced in advance into the supporting medium and the method is applicable to various types of supporting mediums.

When the graft chain is introduced by the "radiation graft polymerization method", any means can be employed for generating radicals from the supporting medium; however, to uniformly generate radicals from the whole supporting medium, irradiation of ionizing radiation is preferable. Examples of the ionizing radiation include a γ ray, an electron beam, a β ray and a neutron beam. For irradiation of ionizing radiation on an industrial scale, an electron beam or a γ ray is preferable. Ionizing radiation can be obtained from a radioactive isotope such as cobalt 60, strontium 90 and cesium 137 or from an X-ray imager, an electron beam accelerator and a UV ray irradiation apparatus and others.

The irradiation dose of ionizing radiation is preferably 1 kGy or more and 1000 kGy or less, more preferably 2 kGy or more and 500 kGy or less and further preferably 5 kGy or more and 200 kGy or less. If the irradiation dose is less than 1 kGy, it tends to be difficult to generate radicals uniformly. In contrast, if the irradiation dose exceeds 1000 kGy, the physical strength of the supporting medium tends to decrease.

Graft polymerization methods using irradiation of ionizing radiation are generally classified roughly into a pre-irradiation method, in which radicals are generated from the supporting medium and then allowed to be in contact with a reactive compound, and a simultaneous irradiation method, in which radicals are generated from the supporting medium in contact with a reactive compound. In the embodiment, either method can be applied; however, the pre-irradiation method manufacturing the small amount of oligomers is preferable.

In the embodiment, the solvent to be used in graft polymerization is not particularly limited as long as it can homogeneously dissolve a reactive compound. As such a solvent, an alcohol such as ethanol, isopropanol and t-butyl alcohol; an ether such as diethyl ether and tetrahydrofuran, a ketone such as acetone and 2-butanone, water or a mixture of these can be used.

In the embodiment, examples of the monomer having the coupling group to be used in graft polymerization include monomers such as acrylic acid and methacrylic acid when a carboxyl group is used as the coupling group. When a primary amino group is used as the coupling group, e.g., allyl amine can be used. When an epoxy group is used as the coupling group, e.g., glycidyl methacrylate can be used.

In the embodiment, a monomer having a precursor functional group which can be converted into the coupling group is grafted to the supporting medium and thereafter the precursor functional group grafted may be converted into the coupling group. The glycidyl methacrylate (GMA) having an epoxy group can be converted into various functional groups by use of various ring-opening reactions of the epoxy group and thus can be preferably used also industrially.

When a carboxyl group is used as the coupling group, ring-opening half esterification reaction may be employed, in which first, GMA is graft polymerized, and thereafter, the epoxy group of the GMA is hydrolyzed into a diol, a cyclic acid anhydride is reacted with the hydroxide group derived from diol through a ring-opening half esterification reaction to form a carboxyl group derived from the cyclic acid anhydride. In view of manufacturing cost, the cyclic acid anhydride is desirably succinic anhydride or glutaric anhydride, but not limited to these.

The catalyst to be used in the ring-opening half esterification reaction is not particularly limited as long as it accelerates the reaction. Specific examples thereof include triethylamine, isobutyl ethylamine, pyridine and 4-dimethylaminopyridine. Triethylamine or 4-dimethylaminopyridine is preferable. In view of the reaction rate and yield, 4-dimethylaminopyridine is the most preferable.

The ring-opening half esterification reaction is preferably performed in an inert organic solvent such as toluene having the above catalyst added therein.

In the embodiment, the NHS activation reaction refers to a step of converting the carboxyl group formed by the above ring-opening half esterification reaction into an active ester. The active ester has a high reactivity compared to the carboxyl group. Therefore, if temperature responsive protein A is desired to be quickly immobilized on the carrier, an active esterification step is preferably employed.

The active ester plays a role in connecting a hydrophilic compound to an immobilization target substance via a covalent bond. The active ester herein refers to a chemical structure represented by R-C(=O)-X. X corresponds to a leaving group such as halogen, a N-hydroxysuccinimide group or a derivative thereof, a 1-hydroxybenzotriazole group or a derivative thereof, a pentafluorophenyl group and a para-nitrophenyl group, but is not limited to these. As the active ester, a N-hydroxysuccinimide ester is desirable in view of reactivity, safety and manufacturing cost. Conversion of a carboxyl group into a N-hydroxysuccinimide ester is carried out by reacting N-hydroxysuccinimide and a carbodiimide at the same time with a carboxyl group. The carbodiimide herein refers to an organic compound having a chemical structure represented by -N=C=N-. Examples thereof include dicyclohexyl carbodiimide, diisopropyl carbodiimide and 1-ethyl-3-(3-dimethylaminopropyl) carbodiimide hydrochloride, but are not limited to these. The concentrations of N-hydroxysuccinimide and carbodiimide are each desirably set at 1 to 100 mmol/L and the reaction temperature at 0 to 100°C, reaction time within a range of 2 minutes to 16 hours. As a reaction solvent, N, N'-dimethylformamide (DMF) and toluene can be used.

In the embodiment, temperature responsive protein A, which is protein A mutated such that its binding property to an antibody changes depending upon temperature, can be prepared with reference to Patent Literature (WO2008/143199).

In the embodiment, the coupling reaction between an NHS activated carboxyl group and temperature responsive protein A is performed, for example, as follows. First, a 0.1 to 100 mg/mL temperature responsive protein A solution is prepared by using a buffer containing no amino group component such as a citric acid buffer (pH 3.0 to 6.2), an acetic acid buffer (pH 3.6 to 5.6), phosphate buffered saline (PBS, pH 5.8 to 8.5) or a carbonate buffer (pH 9.2 to 10.6). The aqueous solution is contacted with the surface of an active ester. Consequently, a functional group such as an amino group contained in temperature responsive protein A reacts with an active ester to form an amide bond. As a result, temperature responsive protein A is immobilized to the surface via a covalent bond. The contact time herein may be set within the range of 2 minutes to 16 hours. After temperature responsive protein A is immobilized, the carrier is desirably washed with an appropriate washing liquid. The washing liquid herein is desirably a buffer containing about 0.5 mol/L salt (NaCl) and an about 0.1% nonionic surfactant. This is because temperature responsive protein A not covalently bonded and just physically adsorbed can be eliminated in this manner.

In the embodiment, the coupling reaction between the NHS activated carboxyl group and temperature responsive  protein A is performed at a temperature at which temperature responsive protein A has adsorptivity to an antibody. In a part of temperature responsive protein A, loss and formation of a native configuration reversibly occurs. To describe more specifically, a part of temperature responsive protein A has a binding capacity to an antibody in a low temperature region where a native configuration is formed; however, in a high temperature region, the binding capacity to the antibody significantly decreases due to loss of the native configuration. If coupling with the supporting medium is made in the low temperature region where a native configuration is formed, temperature responsive protein A maintains a binding property to an antibody and loss and formation of a native configuration by temperature change reversibly occurs after coupling with the supporting medium. However, if coupling with the supporting medium is made in the high temperature region where a native configuration is impaired, temperature responsive protein A immobilized cannot regain a complete native configuration even if the ambient temperature changes to a low temperature, with the result that sufficient antibody binding property cannot be expressed.

Note that a mechanism of changing the binding property of temperature responsive protein A to an antibody depending upon the temperature is not limited to  configuration change. There is temperature responsive protein A, which changes a binding property to an antibody depending upon temperature without configuration change. The temperature responsive protein A, which changes a binding property to an antibody depending upon temperature without configuration change can also maintain the binding property to an antibody after coupling by performing a coupling reaction with an NHS activated carboxyl group at a temperature at which temperature responsive protein A has adsorptivity to the antibody.

In the embodiment, the "first temperature region" or "temperature region A" refers to a temperature region in which temperature responsive protein A has adsorptivity to a specific protein and in which a protein binding amount relative to the maximum binding amount of protein capable of binding to a predetermined amount of temperature responsive protein A becomes 50% or more. The "temperature of the first temperature region" is preferably 0 to less than 30°C, further preferably 0 to 20°C, and most preferably 0 to 10°C. If the temperature is less than 0°C, freezing may occur during a coupling reaction. If the temperature is 30°C or more, it is difficult to obtain a sufficient binding property to an antibody. Furthermore, in the embodiment, the "second temperature region" or "temperature region B" refers to a temperature region in which temperature responsive protein A does not exhibit effective adsorptivity to a specific protein and in which a protein binding amount relative to the maximum binding amount of protein capable of binding to a predetermined amount of temperature responsive protein A becomes less than 50%.

A method for specifically determining temperature region A and temperature region B is carried out in accordance with the following procedure:

1. To a temperature responsive protein A immobilized carrier, a protein is allowed to bind separately at less than 5°C and 10°C (hereinafter, temperatures are set at intervals of 10°C up to a temperature immediately below the denaturation temperature of a specific protein).

2. The protein bound is eluted by increasing the temperature up to a temperature immediately below the denaturation temperature of the specific protein. The elution amount of protein is measured at by ultraviolet ray (UV) method at 280 nm.

3. The elution amounts of protein are plotted against the temperatures at which the protein is adsorbed. An intersection between the line corresponding to 50% of the maximum value of the protein binding amount (elution amount) and the line connecting plots is obtained (hereinafter referred to as "50%-binding temperatures"). Using the intersection as a boarder, the temperature region where a protein binding amount exhibits 50% or more is defined as temperature region A, whereas the temperature region where a protein binding amount exhibits less than 50% is defined as temperature region B.

Temperature responsive protein A prepared with reference to Example of Patent Literature (WO2008/143199) was immobilized in accordance with the method described in Example 1 (described later). Then, an antibody was adsorbed and eluted in the conditions described in Example 1 and 50%-binding temperature was calculated in accordance with the aforementioned procedure. As a result, as shown in Figure 1, a predetermined amount of temperature responsive protein A can be bound to 14.5 mg/mL of antibody at an ambient temperature (antibody binding temperature) of 2°C and the binding amount at 2°C was maximum. Furthermore, the 50% adsorption temperature was found to be 15.0°C based on plotting. In this case, the coupling temperatures of 15.0°C or less falls within the aforementioned temperature region A, the coupling temperatures higher than 15.0°C falls within the temperature region B.

Note that the temperature, at which temperature region A of temperature responsive protein A is partitioned from temperature region B, slightly varies depending upon the difference in the peptide chain of temperature responsive protein A. However, if temperature responsive proteins A have the same peptide chain, the temperature, at which temperature region A of temperature responsive protein A is partitioned from temperature region B, does not change. What is changed is an absolute binding amount of adsorbable and desorbable antibody.

After temperature responsive protein A is immobilized to the carrier surface (preferably, further after the temperature responsive protein A immobilized carrier is washed), an unreacted carboxyl group or active ester is preferably bound to a low molecular compound having an amino group to thereby convert the carboxyl group or active ester into a functional group having a lower reactivity. In this manner, undesirable immobilization of unwanted molecules such as impurities to the carrier surface can be prevented. This operation is preferably performed, particularly in the case where the terminal functional group of temperature responsive protein A immobilized carrier is an active ester.

In the specification, the operation for reacting a low molecular compound having an amino group with an active ester group is particularly described often as "blocking". However, the surface of the carrier after the carboxyl group or the active ester is reacted with the low molecular compound is desirably hydrophilic. This is because the hydrophilic surface generally has an effect of suppressing nonspecific adsorption of a bio-related substance. To attain this, a low molecular compound further having a hydrophilic group other than the amino group is preferably used as the low molecular compound having an amino group. Non-limiting examples of such a low molecular compound include ethanolamine, trishydroxymethylaminomethane, and diglycolamine (IUPAC name: 2-(2-aminoethoxy) ethanol). These low molecular compounds are each dissolved in a buffer such as PBS so as to obtain 10 to 1, 000 mmol/L. The solution is contacted with the carrier having temperature responsive protein A immobilized thereto. For example, the reaction temperature is 4 to 37°C, the reaction time may be set within the range of 2 minutes to 16 hours.

The temperature responsive protein A immobilized carrier employs a neutral solution having pH within the range of 4 to 8 as a preservation solution and stored at a temperature as low as about 2 to 10°C. As the preservation solution, 20% ethanol is preferable in consideration of antibacterial activity.

A method for separating an antibody by the temperature responsive protein A immobilized carrier is not particularly limited. For example, a method in which the temperature at which the characteristics of a temperature responsive protein A are changed is previously checked and impurities are separated by changing temperature with the predetermined temperature sandwiched in the middle can be used. In the case of the embodiment, the method is most effectively used if impurities are separated by changing temperature with the predetermined temperature, at which the characteristics of the temperature responsive protein A are greatly changed, sandwiched in the middle.

In the temperature responsive protein A immobilized carrier manufactured by the temperature responsive protein A immobilization method according to the embodiment described above, the antibody binding property of protein A is maintained after it is immobilized. Thus, an industrially sufficient amount of antibody can be purified by temperature change. In this case, separation can be made by a simple operation i.e., just by changing the temperature within a column. In addition, since no eluate of low pH is required in an elution step, an antibody can be purified without being denatured.

Example 1

The embodiment will be more specifically described below based on examples; however, these examples should not be construed as limiting the embodiment.

A graft chain formed of a coupling-group precursor monomer, i.e., glycidyl methacrylate (GMA), by a radiation graft polymerization method was introduced in a hollow fiber membrane, and thereafter, an epoxy group contained in GMA was converted into a diol group, and then, converted into a carboxyl group. Subsequently, the hollow fiber membrane in which the graft chain having the carboxyl group in a side chain was introduced was molded into a module and thereafter, the carboxyl group is activated by NHS. Furthermore, through the NHS activated hollow fiber module, temperature responsive protein A is passed at a temperature at which temperature responsive protein A has an adsorptivity to an antibody to immobilize temperature responsive protein A to the hollow fiber. This will be more specifically described as follows.

1) Surface graft polymerization

GMA (20 g) was dissolved in methanol (180 mL) and bubbled with nitrogen for 30 minutes. This was used as a reaction solution. Two grams of a hollow fiber (inner diameter: 2.0 mm, outer diameter: 3.0 mm, average pore diameter: 0.25 $\mu$m) made of polyethylene was cooled with dry ice to -60°C under a nitrogen atmosphere, and irradiated with $\gamma$

rays (200 kGy) using cobalt (Co) 60 as a radiation source. The hollow fiber irradiated was allowed to stand still under a reduced pressure of 13.4 Pa or less for 5 minutes and thereafter contacted with the above reaction solution (20 mL) at 40°C and allowed to stand still for 16 hours. Thereafter, the hollow fiber was washed with ethanol and dried in a vacuum dryer.

2) Conversion of epoxy group to diol

The hollow fiber polymerized by surface graft polymerization was placed in a 0.5 mol/L sulfuric acid and reacted at 80°C for 2 hours, to convert the remaining epoxy group in the graft chain into a diol group. After completion of the reaction, the hollow fiber was washed with pure water, and a membrane was washed with ethanol and dried in a vacuum drier.

3) Introduction of carboxyl group

The hollow fiber having the diol group converted from the epoxy group was soaked in a reaction solution prepared by dissolving succinic anhydride (3.0 g) and 4-dimethylaminopyridine (3.6 g) in a toluene (900 mL) and reacted at 40°C for 60 minutes to introduce a carboxyl group into the graft chain. After completion of the reaction, the hollow fiber was washed with ethanol and dried in a vacuum dryer.

4) Activation by NHS

While the modulated hollow fiber (a single hollow fiber module, effective fiber length: 4 cm) was warmed to 40°C, an NHS activation reaction solution (NHS (0.07 g), dehydrated isopropyl alcohol (45 mL), diisopropyl carbodiimide (0.09 mL)) was passed through at a flow rate of 0.4 mL/minute for 60 minutes to activate the carboxyl group by NHS. After completion of the reaction, while the hollow fiber module was ice cooled, dehydrated isopropyl alcohol was passed through the hollow fiber module at a flow rate of 0.4 mL/minute for 60 minutes to wash the hollow fiber module. The hollow fiber module washed was stored at 4°C with dehydrated isopropyl alcohol stored therein.

5) Coupling of temperature responsive protein A

Temperature responsive protein A was prepared with reference to Example of Patent Literature (WO2008/143199) and put in use. Though the hollow fiber module having the carboxyl group activated by NHS, an ice cooled 1 mmol/L hydrochloric acid (10 mL) was passed to replace the dehydrated isopropyl alcohol serving as a preservation solution. Subsequently, temperature responsive protein A (20 mg) was dissolved in 7 mL of a coupling buffer (0.2 mol/L phosphate buffer, 0.5 mol/L NaCl, pH 8.3), cooled to 2°C and passed through the follow fiber module at a flow rate of 0.4 mL/minute. The solution thus passed though was continuously added to a supply solution and circulated in this manner for 16 hours. The module was kept at 2°C during the circulation to keep the temperature in the coupling process at 2°C. After a predetermined time, the coupling buffer was allowed to pass through the hollow fiber module to wash and collect the temperature responsive protein A not coupled with the NHS activated group.

6) Blocking

Through the hollow fiber module coupled with temperature responsive protein A, 10 mL of a blocking reaction solution (0.5 mol/L ethanolamine, 0.5 mol/L NaCl, pH 8.0) was passed and allowed to stand still at room temperature for 30 minutes to block the remaining NHS with ethanolamine. After completion of the reaction, the hollow fiber module was washed with pure water and then stored at 4°C with 20% ethanol stored therein.

7) Measurement of elution amount of antibody by temperature change

Using a chromatography system (AKTA FPLC, manufactured by GE Healthcare Japan), an adsorption/elution test of an antibody (Venoglobulin-IH blood donation, manufactured by Benesis Corporation) by temperature change was performed. An operation for changing the temperature of the hollow fiber module was performed by temporarily stopping the pump of the chromatography system, soaking the hollow fiber module in a constant-temperature water vessel set at a predetermined temperature, and thereafter storing it in a warm place for 10 minutes or more, and driving the pump of the chromatography system, again. Adsorption and elution of the antibody were performed in the following conditions.

(Adsorption step)

- An antibody concentration: 2.5 mg/mL
- Adsorption buffer: 20 mmol/L phosphate buffer, 150 mmol/L NaCl (pH 8.0)
- An antibody loading amount: 20 mL
- Flow rate: 0.4 mL/min
- Hollow fiber membrane volume: 0.18 mL
- Adsorption (coupling) temperature: 2°C (Washing step)
- Wash buffer: the same as the adsorption buffer
- Flow rate: 0.4 mL/min
- Washing temperature: 2°C

(Elution step by temperature change)

- Buffer for elution by temperature change: the same as the adsorption buffer
- Flow rate: 0.4 mL/min
- Amount of liquid passed through: 20 mL
- Elution temperature: 40°C

(Low pH elution step after elution step by temperature change)

- Low pH elution buffer: 100 mmol/L, citric acid buffer (pH 3.0)
- Flow rate: 0.4 mL/min
- Amount of liquid passed through: 20 mL
- Elution temperature: 40°C

After elution by temperature change, the antibody that cannot be completely eluted by temperature change was eluted with a Low pH elution buffer. UV absorption (280 nm) of fractions in each step was measured and an immunoglobulin concentration was calculated in accordance with the following expression to obtain the elution amount of immunoglobulin by temperature change.

$$\text{An immunoglobulin concentration (mg/mL)} = \text{absorbance at 280 nm}/14 \times 10$$

$$\text{Elution amount by temperature change (mg/mL)} = \text{immunoglobulin concentration of fraction eluted by temperature change} \times \text{liquid amount of fraction eluted by temperature change}/\text{membrane volume}$$

(Results)

The density of the coupling group in the hollow fiber membrane was measured by a gravimetric method. As a result, the density was 160 µmol/mL. The elution  amount of antibody by temperature change was 14.5 mg/mL-membrane volume. It was demonstrated that the antibody can be eluted by temperature change. After elution by temperature change, the antibody left on the hollow fiber was eluted by a low pH elution buffer. As a result, the elution amount was as low as 1.2 mg/mL- membrane volume. From the above results, it was demonstrated that the temperature responsive protein A immobilized hollow fiber can be used for industrial antibody purification.

Example 2

A temperature responsive protein A immobilized hollow fiber was manufactured in the same manner as in Example 1 except that the coupling temperature of temperature responsive protein A to a hollow fiber membrane was set at 10°C.

(Results)

The elusion amount of antibody by temperature change was 14.1 mg/mL-membrane volume, demonstrating that the antibody can be eluted by temperature change. After elution by temperature change, the antibody left on the hollow fiber was eluted by a low pH elution buffer. As a result, the elution amount was as low as 1.2 mg/mL-membrane volume. From the above results, it was demonstrated that the temperature responsive protein A  immobilized hollow fiber can be used for industrial antibody purification.

Example 3

After the carboxyl group was introduced into the crosslinked polyvinyl alcohol beads, the carboxyl group was activated by NHS. Furthermore, NHS activated crosslinked polyvinyl alcohol beads were contacted with temperature responsive protein A at a temperature at which temperature responsive protein A has adsorptivity to the antibody to immobilize temperature responsive protein A to crosslinked polyvinyl alcohol beads. This will be more specifically described as follows.

1) Introduction of carboxyl group

Succinic anhydride (3.0 g) and 4-dimethylaminopyridine (3.6 g) were dissolved in toluene (450 mL) and used as the reaction solution. One gram of crosslinked polyvinyl alcohol (average particle diameter: 100 $\mu$m) was contacted with the above reaction solution at 50°C and stirred for 2 hours. Thereafter, the crosslinked polyvinyl alcohol beads were washed with dehydrated isopropyl alcohol.

2) Column Packing

The above crosslinked polyvinyl alcohol beads were packed in a vacant column (Tricorn 5/20 column, manufactured by GE Healthcare Japan).

3) Activation by NHS

While the above column was warmed to 40°C, an NHS activation reaction solution (NHS: 0.07g, dehydrated isopropyl alcohol: 45 mL, diisopropyl carbodiimide: 0.09 mL) was passed through at a flow rate of 0.4 mL/minute for 30 minutes to activate the carboxyl group by NHS. After completion of the reaction, while the column was ice cooled, dehydrated isopropyl alcohol was passed through the column at a flow rate of 0.4 mL/minute for 30 minutes to wash the column. The column washed was stored at 4°C with dehydrated isopropyl alcohol stored therein.

4) Coupling of temperature responsive protein A

Temperature responsive protein A was prepared with reference to Example of Patent Literature (WO2008/143199) and put in use. Though the column having the carboxyl group activated by NHS, an ice cooled 1 mmol/L hydrochloric acid (2 mL) was passed to replace the dehydrated isopropyl alcohol serving as a preservation solution. Subsequently, temperature responsive protein A (30 mg) was dissolved in 1 mL of a coupling buffer (0.2 mol/L phosphate buffer, 0.5 mol/L NaCl, pH 8.3), cooled to 2°C, supplied to the column at a flow rate of 0.4  mL/minute, and stored for 16 hours. The column was kept at 2°C during the storage to keep the temperature in the coupling process at 2°C. After a predetermined time, the coupling buffer was allowed to pass through the column to wash and collect the temperature responsive protein A not coupled with the NHS active group.

5) Blocking

Through the column coupled with temperature responsive protein A, 10 mL of a blocking reaction solution (0.5 mol/L ethanolamine, 0.5 mol/ L NaCl, pH 8.0) was passed and allowed to stand still at room temperature for 30 minutes to block the remaining NHS with ethanolamine. After completion of the reaction, the column was washed with pure water and then stored at 4°C with 20% ethanol stored therein.

6) Measurement of elution amount of antibody by temperature change

Using a chromatography system (AKTA FPLC, manufactured by GE Healthcare Japan), an adsorption/elution test of an antibody (Venogloblin-IH blood donation, manufactured by Benesis Corporation) by temperature change was performed. An operation for changing the temperature of the column was performed by temporarily stopping the pump of the chromatography system, soaking the column in a constant-temperature  water vessel set at a predetermined temperature, and thereafter storing it in a warm place for 10 minutes or more and driving the pump of the chromatography system, again. Adsorption and elution of the antibody were performed in the following conditions.

(Adsorption step)

- An antibody concentration: 2.5 mg/mL
- Adsorption buffer: 20 mmol/L phosphate buffer, 150 mmol/L NaCl (pH 8.0)
- An antibody loading amount: 20 mL
- Flow rate: 0.4 mL/min
- Bead volume: 0.59 mL
- Adsorption (coupling) temperature: 2°C

(Washing step)

- Wash buffer: the same as the adsorption buffer
- Flow rate: 0.4 mL/min
- Washing temperature: 2°C

(Elution step by temperature change)

- Elution buffer: the same as the adsorption buffer
- Flow rate: 0.4 mL/min
- Amount of liquid passed through: 20 mL
- Elution temperature: 40°C

(Low pH elution step after elution step by temperature change)

- Low pH elution buffer: 100 mmol/L citric acid buffer (pH 3.0)
- Flow rate: 0.4 mL/min
- Amount of liquid passed through: 20 mL
- Elution temperature: 40°C

After elution by temperature change, an antibody that cannot be completely eluted by temperature change was eluted with a Low pH elution buffer. UV adsorption (280 nm) of fractions in each step was measured and an immunoglobulin concentration was calculated in accordance with the following expression to obtain the elution amount of immunoglobulin by temperature change.

An immunoglobulin concentration (mg/mL) = absorbance at 280 nm/14 × 10

Elution amount by temperature change (mg/mL) = immunoglobulin concentration of fraction eluted by temperature change × liquid amount of fraction eluted by temperature change/bead volume

(Results)

The density of the coupling groups in the beads was measured by the HPLC method. As a result, the density was 59 μmol/mL. The elution amount of antibody by temperature change was 32.1 mg/mL-bead volume. It was demonstrated that the antibody can be eluted by temperature change. After elution by temperature change, the antibody left on the beads was eluted by a low pH elution buffer. As a result, the elution amount was as low as 0.3 mg/mL-bead volume. From the above results, it was demonstrated that the temperature responsive protein A immobilized beads can be used for industrial antibody purification.

Example 4

Temperature responsive protein A immobilized beads were manufactured in the same manner as in Example 1 except that cellulose beads (manufactured by Chisso Corporation) were used as the carrier.

(Results)

The density of the coupling groups in the beads was measured by the HPLC method. As a result, the density was 59 μmol/mL. The elution amount of the antibody by temperature change was 18.9 mg/mL- bead volume. It was demonstrated that the antibody can be eluted by temperature change. After elution by temperature change, the antibody left on the beads was eluted by a low pH elution buffer. As a result, the elution amount was as low as 0.4 mg/mL-bead volume. From the above results, it was demonstrated that the temperature responsive protein A immobilized beads can be used for industrial antibody purification.

Example 5

Crosslinked cellulose beads (trade name: Cellufine Formyl, product number: 676944324, manufactured by Chisso Corporation) having a formyl group (aldehyde group) as the coupling group in an amount of 10 μmol/mL (catalog value) were used.

1) Coupling with temperature responsive protein A

First, temperature responsive protein A (20 mg) was dissolved in 2 mL of a coupling buffer (0.2M phosphate buffer, pH 8.3). Four milliliters of Cellufine Formyl (50% slurry) was washed with pure water, mixed with the above solution and shaken in a constant-temperature shaker at 2°C for 2 hours. Thereafter, 18 mg of a reducing agent (trimethylamine borane) was added and reacted in a constant-temperature shaker at 2°C for 4 hours. After a predetermined time, the beads were washed with the above coupling buffer. To the beads, 4 mL of a blocking liquid (0.2M tris hydrochloric acid buffer) and 15 mg of a reducing agent (trimethylamine borane) were added and reacted in a constant-temperature shaker at 2°C for 2 hours. After a predetermined time, the beads were washed with pure water.

2) Measurement of elution amount of antibody by temperature change

The elution amount of antibody by temperature change was measured in the same manner as in Example 1.

(Results)

The elution amount of the antibody by temperature change was 21.1 mg/mL-bead volume. It was demonstrated that the antibody can be eluted by temperature change. After elution by temperature change, the antibody left on the beads was eluted by a low pH elution buffer. As a result, the elution amount was as low as 1.2 mg/mL-bead volume. From the above results, it was demonstrated that the temperature responsive protein A immobilized beads can be used in industrial antibody purification.

The results of Examples 1 to 5 described above are shown in Table 1.

[Table 1]

|  | Example 1 | Example 2 | Example 3 | Example 4 | Example 5 |
|---|---|---|---|---|---|
| Carrier | PE hollow fiber | PE hollow fiber | Crosslinked polyvinyl alcohol beads | Crosslinked cellulose beads | Crosslinked cellulose beads (Cellufine Formyl) |
| Coupling temperature °c | 2 | 10 | 2 | 2 | 2 |
| Elution amount of antibody by temperature change mg/mL | 14.5 | 14.1 | 32.1 | 18.9 | 21.1 |

[Comparative Example 1]

Temperature responsive protein A immobilized hollow fiber was manufactured in the same manner as in Example 1 except that the coupling temperature of temperature responsive protein A to the hollow fiber membrane was set at 20°C.

(Results)

The elution amount of the antibody by temperature change was 6.1 mg/mL-membrane volume. After elution by temperature change, the antibody left on the hollow fiber was eluted by a low pH elution buffer. As a result, the elution amount was 1.1 mg/mL-membrane volume.

[Comparative Example 2]

Temperature responsive protein A immobilized hollow fiber was manufactured in the same manner as in Example 1 except that the coupling temperature of temperature responsive protein A to the hollow fiber membrane was set at 30°C.

(Results)

The elution amount of the antibody by temperature change was 5.4 mg/mL-membrane volume. After elution by temperature change, the antibody left on the hollow fiber was eluted by a low pH elution buffer. As a result, the elution amount was 1.1 mg/mL-membrane volume.

[Comparative Example 3]

Temperature responsive protein A immobilized beads were manufactured in the same manner as in Example 3 except that the coupling temperature of temperature responsive protein A to the crosslinked polyvinyl alcohol beads was set at 30°C.

(Results)

The elution amount of the antibody by temperature change was 26.1 mg/mL-bead volume. After elution by temperature change, the antibody left on the beads was eluted by a low pH elution buffer. As a result, the elution amount was 0.3 mg/mL-bead volume.

[Comparative Example 4]

Temperature responsive protein A immobilized beads were manufactured in the same manner as in Example 4 except that the coupling temperature of temperature responsive protein A to the crosslinked cellulose beads was set at 30°C.

(Results)

The elution amount of the antibody by temperature change was 14.2 mg/mL-bead volume. After elution by temperature change, the antibody left on the beads was eluted by a low pH elution buffer. As a result, the elution amount was 0.3 mg/mL-bead volume.

[Comparative Example 5]

Temperature responsive protein A immobilized beads were manufactured in the same manner as in Example 4 except that the coupling temperature of temperature responsive protein A to the Cellufine Formyl was set at 30°C.

(Results)

The elution amount of the antibody by temperature change was 17.1 mg/mL-bead volume. After elution by temperature change, the antibody left on the beads was eluted by a low pH elution buffer. As a result, the elution amount was 1.0 mg/mL-bead volume.
The results of Comparative Examples 1 to 5 are shown in Table 2.

[Table 2]

|  | Comparative Example 1 | Comparative Example 2 | Comparative Example 3 | Comparative Example 4 | Comparative Example 5 |
|---|---|---|---|---|---|
| Carrier | PE hollow fiber | PE hollow fiber | Crosslinked polyvinyl alcohol beads | Crosslinked cellulose beads | Crosslinked cellulose beads (Cellufine Formyl) |
| Coupling temperature °c | 20 | 30 | 30 | 30 | 30 |
| Elution amount of antibody by temperature change mg/mL | 6.1 | 5.4 | 26.1 | 14.2 | 17.1 |

The present application was made based on Japanese Patent Application (Japanese Patent Application No. 2010-288451) filed on December 24, 2010 with the Japanese Patent Office and the content thereof is incorporated by reference.

Industrial Applicability

If the method for manufacturing the temperature responsive protein A immobilized carrier according to the embodiment and the temperature responsive protein A immobilized carrier manufactured by the method are used, useful physiologically active compounds such as globulins can be fractionated by temperature change on an industrial scale.

Claims

**1.** A method for manufacturing a temperature responsive protein A immobilized carrier, the temperature responsive protein A being mutated such that a binding property to an antibody changes depending upon temperature, wherein the temperature responsive protein A has the binding property to the antibody in a first temperature region and no binding property to the antibody in a second temperature region; and
the method comprises a step of immobilizing the temperature responsive protein A to a carrier surface at a temperature within the first temperature region in which the temperature responsive protein A has the binding property to the antibody.

2. The method for manufacturing the temperature responsive protein A immobilized carrier according to Claim 1, wherein the temperature responsive protein A is immobilized to the carrier surface by a covalent bond.

3. The method for manufacturing the temperature responsive protein A immobilized carrier according to Claim 1 or 2, wherein the step of immobilizing the temperature responsive protein A to the carrier surface comprises contacting the temperature responsive protein A with a coupling group immobilized to the carrier surface.

4. The method for manufacturing the temperature responsive protein A immobilized carrier according to Claim 3, wherein the concentration of the coupling group immobilized to the carrier surface is 50 $\mu$mol/mL or more.

5. The method for manufacturing the temperature responsive protein A immobilized carrier according to any one of Claims 1 to 4, wherein the carrier is a membrane.

6. The method for manufacturing the temperature responsive protein A immobilized carrier according to Claim 5, wherein the membrane has hollow fiber form.

7. The method for manufacturing the temperature responsive protein A immobilized carrier according to any one of Claims 1 to 4, wherein the carrier comprises beads.

8. The method for manufacturing the temperature responsive protein A immobilized carrier according to Claim 7, wherein the beads are formed of crosslinked polyvinyl alcohol.

9. The method for manufacturing the temperature responsive protein A immobilized carrier according to Claim 7, wherein the beads are formed of crosslinked cellulose.

10. The method for manufacturing the temperature responsive protein A immobilized carrier according to Claim 3, wherein the temperature responsive protein A is contacted with the coupling group immobilized to the carrier surface by contacting a reaction solution containing the temperature responsive protein A with the carrier.

11. The method for manufacturing the temperature responsive protein A immobilized carrier according to Claim 3, wherein
the carrier has a graft polymer chain on the carrier surface; and
the coupling group is present in a side chain of the graft polymer chain.

12. The method for manufacturing the temperature responsive protein A immobilized carrier according to Claim 3, wherein the coupling group is a carboxyl group activated by N-hydroxysuccinimide.

13. A temperature responsive protein A immobilized carrier manufactured by the manufacturing method according to any one of Claims 1 to 12.

14. A temperature responsive protein A immobilized carrier manufactured by the manufacturing method according to any one of Claims 1 to 12, wherein immunoglobulin adsorbed at a temperature at which temperature responsive protein A has adsorptivity to immunoglobulin can be eluted by temperature change.

15. The temperature responsive protein A immobilized carrier according to Claim 14, wherein 80% or more of the immunoglobulin adsorbed can be eluted by the temperature change.

16. The temperature responsive protein A immobilized carrier according to any one of Claims 13 to 15 having membrane form.

17. The temperature responsive protein A immobilized carrier according to any one of Claims 13 to 16 having hollow fiber form.

18. The temperature responsive protein A immobilized carrier according to any one of Claims 13 to 15 having bead form.

# Fig. 1

| Antibody binding temperature °C | 2 | 10 | 20 | 30 |
|---|---|---|---|---|
| Antibody binding amount mg/mL | 14.5 | 13.1 | 1.9 | 1.6 |

| INTERNATIONAL SEARCH REPORT | International application No. |
|---|---|
| | PCT/JP2011/080065 |

A. CLASSIFICATION OF SUBJECT MATTER
*C07K17/00*(2006.01)i, *C07K17/04*(2006.01)i, *C07K17/06*(2006.01)i, *C07K17/12* (2006.01)i, *G01N30/54*(2006.01)n, *G01N30/88*(2006.01)n

According to International Patent Classification (IPC) or to both national classification and IPC

B. FIELDS SEARCHED

Minimum documentation searched (classification system followed by classification symbols)
C07K17/00, C07K17/04, C07K17/06, C07K17/12, G01N30/54, G01N30/88

Documentation searched other than minimum documentation to the extent that such documents are included in the fields searched
Jitsuyo Shinan Koho        1922–1996    Jitsuyo Shinan Toroku Koho    1996–2012
Kokai Jitsuyo Shinan Koho    1971–2012    Toroku Jitsuyo Shinan Koho    1994–2012

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2008/143199 A1 (Nomadic Bioscience Co., Ltd.), 27 November 2008 (27.11.2008), example 7; paragraph [0168] & US 2010/0168395 A1 | 1-7,10,12-18<br>1-18 |
| Y | OSTROVE S., [29] Affinity chromatography: General methods, Methods in Enzymology, 1990, Vol.182, pp.357-371 | 1-18 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 March, 2012 (01.03.12) | 13 March, 2012 (13.03.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005206602 A
- WO 2008143199 A

- JP 2010288451 A